# EUROPEAN PATENT APPLICATION

(11) **EP 1 975 174 A1**
(43) Date of publication of application: **01.10.2008**
(21) Application number: 06841769.0
(22) Date of filing: 27.12.2006
(51) Int. Cl.: C07K 14/47, C12N 15/12, G01N 33/68, C12Q 1/68, A61K 38/17, A61P 37/06

(54) **SELECTIVE PEPTIDES THAT INHIBIT THE BIOLOGICAL ACTIVITY OF CALCINEURIN**

(30) Priority: 29.12.2005 ES 200503237
(71) Applicant: CONSEJO SUPERIOR DE INVESTIGACIONES CIENTIFICAS, 28006 Madrid (ES); CNIC, Fundacion Centro Nacional de Investigaciones Cardiovasculares Carlos III, 28029 Madrid (ES)
(72) Inventor: REDONDO MOYA, Juan Miguel, 28049 MADRID (ES); RODRÍGUEZ MÁRQUEZ, Antonio, 28049 MADRID (ES); MARTÍNEZ MARTÍNEZ, Sara, 28049 MADRID (ES)
(74) Representative: Pons Arino, Angel
(86) International application number: PCT/ES2006/000717
(87) International publication number: WO 2007/074198

(57) **Abstract**

The invention relates to the biotechnology sector involving the area of human health. More specifically, the invention is based on the surprising usefulness of peptides LxVPc1, c3 and c4 as efficient selective inhibitors of the calcineurin signalling pathway (CN) - NFAT and the phosphate activity of CN. Said compounds are useful immunosuppressors and serve as a base for the preparation of therapeutic compositions for the prophylactics and treatment of human diseases associated with T-lymphocyte activation, including, but not limited to, autoimmune diseases, inflammation and allergy or transplant rejections. In addition, said peptides and the associated biological and genetic material can form useful tools for the development of tests that can be used to find compounds that have a selective antagonist activity in relation to CN.

## Description

### FIELD OF THE INVENTION

This invention relates to the biotechnology sector applied to the area of human health, and more specifically, it is based on the surprising usefulness of peptides LxVPc1, c3 and c4 as efficient selective inhibitors of the calcineurin (CN)-NFAT signalling pathway and of the phosphatase activity of CN. Said compounds are useful immunosuppressors and serve as a base for the preparation of therapeutic compositions for the prophylaxis and treatment of human diseases associated with T-lymphocyte activation, such as, but not limited to, autoimmune diseases, inflammation and allergy or transplant rejection situations. Similarly, said peptides and the associated biological and genetic material can form useful tools for the development of tests that can be used to find compounds that have a selective antagonist activity in relation to CN.

### BACKGROUND OF THE INVENTION

The Calcium/Calcineurin/NFAT pathway plays a prominent role in a large number of important biological processes, including the development and functioning of the immune and nervous systems, formation of the vasculature, morphogenesis of the cardiac valves and muscular development and growth (1-5). The calcium signals activate the phosphatase calcineurin (CN), which dephosphorylates the NFATc1, NFATc2, NFATc3 and NFATc4 transcription factors (HUGO nomenclature). This brings about their translocation from the cytoplasm to the nucleus and increases their capacity of binding with the DNA, causing the gene expression which is dependent on NFAT (6). The binding of CN to the NFATs is therefore an essential step in the activation of this family of transcription factors.

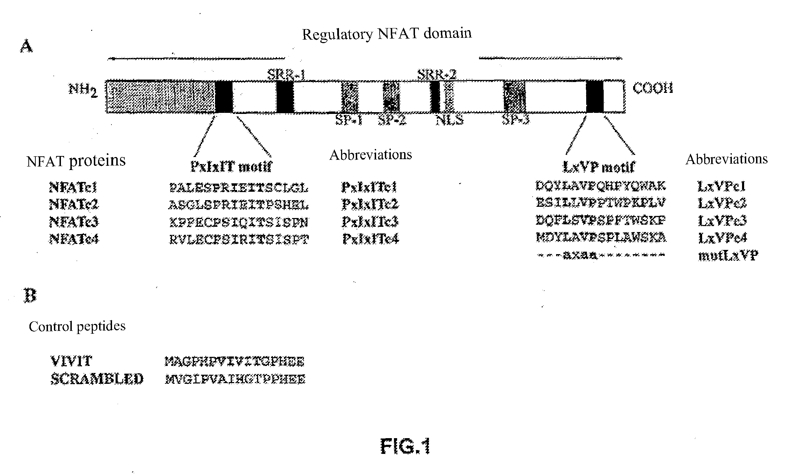

Calcineurin, also known as the phosphatase 2B protein, is a heterodimer which consists of a catalytic sub-unit (CnA) tightly bound to a regulatory sub-unit (CnB). CnA is comprised of a catalytic domain and another regulatory domain which contains the CnB binding sequence, a calmodulin (CaM) binding domain and an autoinhibitory segment (7). The phosphatase activity of CnA is regulated by a complex formed by Ca²⁺, CnB and CaM; and both regulatory proteins are essential for this activity (7, 8). CN can bind both to phosphorylated and to dephosphorylated NFATs (9, 10), and although non-active CN can bind to NFATs, the binding is stronger with activated CN (11). In NFATs, this interaction has been mapped within the N-terminal regulatory domain, which is conserved in all four members (12-14). By means of mutational analysis, the motifs conserved within the regulatory domain of NFATc2 were analysed, defining the sequence SPRIEIT as a calcineurin docking site (15). In subsequent studies, the residues conserved in the corresponding sequences of other NFAT members were identified, defining the PxlxlT motif (see Figure 1a), which appears to be functional in all the CN-regulated NFAT members (11, 16-18).

NFAT was first identified as a factor essential for IL-2 gene expression (19), and was subsequently implicated in the regulation of many other genes necessary for an effective immune response (20-23). The discovery that CN is the intracellular target of immunosuppressor drugs such as cyclosporin A (CsA) and FK506 (24) further highlighted the importance of NFAT in the immune system and also in the Ca²⁺/CN/NFAT pathway (25-27). The use of these drugs, CsA and FK506, is the most common method for blocking the CN-NFAT signalling pathway, as they inhibit the enzymatic activity of CN (24). However, this strategy also inhibits other CN-dependent pathways unrelated to NFAT, which is associated with the severe side-effects which its clinical use causes (28). In addition, due to the interaction of these drugs with their corresponding intracellular partners (known as immunophilins), non-CN-dependent cell processes could be responsible for some of the non-desire effects associated with their administration.

It has been seen by means of studies using inhibiting peptides that the specific blocking of protein-protein interactions is therapeutically promising for the treatment of diseases in which the CN-NFAT pathway is involved. The VIVIT peptide, whose sequence is based on the PxlxlT motif and which features a high affinity for CN, is capable of selectively inhibiting the NFAT-dependent signalling pathway without affecting the rest of the CN-activated pathways (29). However, over the last years, numerous CN-binding proteins have been described, the majority of which present a motif similar to that of PxlxlT within the CN-interaction zone (30). The peptide VIVIT might therefore block both the CN-NFAT interaction and that of CN with other proteins. On the other hand, the LxVP motif -which takes part in the interaction of NFAT with activated CN- has only been described in NFAT. Therefore, the blocking effect, both *in vitro* and *in vivo* described herein shows that the use of peptides derived from these motifs has a potential selective inhibitory effect on the CN-NFAT pathway, thus fulfilling the need to find highly selective molecules capable of inhibiting the CN-NFAT pathway.

### DESCRIPTION OF THE INVENTION

### BRIEF DESCRIPTION OF THE INVENTION

This invention relates to new peptides and to derivatives of the same which are useful as selective antagonists (inhibitors) of the CN-NFAT signalling pathway, and more specifically, as immunosuppressors (furthermore, it has been observed that they are inhibitors of CN phosphatase activity). The invention also relates to therapeutic compounds comprising said peptides, to screening assays of compounds which have preferably selective antagonistic activity on the biological action of CN on NFAT, and the use of said peptides for the prophylaxis and treatment of human diseases associated with T-lymphocyte activation such as, but not limited to, autoimmune diseases, inflammation and allergy, or transplant rejection situations. In the same way, it relates to genetic tools and methods for the production of said peptides.

### DESCRIPTION OF THE FIGURES

Figure 1A:- A schematic representation of the NH₂-terminal regulatory domain and of the CN-interacting sequences of the different NFATs. The conserved regions among the different NFAT members are portrayed as follows: SP-1, SP-2 and SP-3 are serine-proline repeat motifs, susceptible to phosphorylation; SRR-1 and SRR-2 are serine-rich regions, and NLS is the nuclear localisation sequence. The two regions involved in binding CN, labelled PxlxlT and LxVP motifs, are portrayed in black, and the sequences corresponding to these motifs in each of the four human NFAT members are portrayed below; the amino acids conserved among the various members are portrayed in bold type. The residues shown were replaced by alanines in order to generate the corresponding mutant LxVPx1 peptide. The abbreviations used for the different PxlxlT and LxVP motifs are portrayed on the right.
Figure 1B:- Amino acid sequences of the peptides used as positive (VIVIT) or negative (SCRAMBLED) competitive controls for the *in vitro* binding experiments.

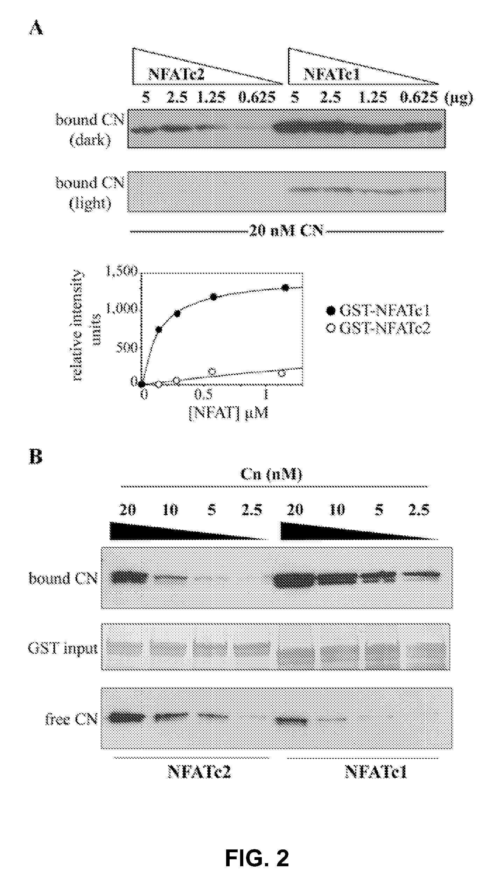

Figure 2:- Comparison of CN binding to NFATc1 and NFATc2 *in vitro.* Figure 2A: The binding of NFATc1 and NFATc2 to CN is dependent on the NFAT concentration. A fixed concentration of CN (20nM) was assayed with decreasing concentrations of the regulatory domain of NFATc2 or NFATc1 fused to GST (*Glutathione-S-Transferase*) in pull-down tests. Bound CN was visualised by Western Blot using a monoclonal anti-CnA antibody. Because of the high amounts of CN bound to NFATc1, two different exposures are shown (top and bottom panels respectively) to show clearly the NFATc1-CN interaction. Bound CN was quantified in a densitometer, and these data, as relative intensity units of CN bound at each NFAT concentration, are presented in the bottom graphic. Figure 2B: The CN-NFAT binding is dependent on CN concentration. Fixed quantities of GST-NFATc2 (left) or GST-NFATc1 (right) were assayed with decreasing concentrations of CN (20, 10, 5 and 2.5 nM of CN). CN was detected using an anti-CnA monoclonal antibody; bound CN is portrayed in the upper panel; in the lower panel, the unbound (free) CN in one-third volumes of the supernatants produced after the binding reaction. *Ponceau red* staining of the *Western blot* membranes confirmed the use of equal quantities of GST fusion proteins in the reactions (see middle panel).

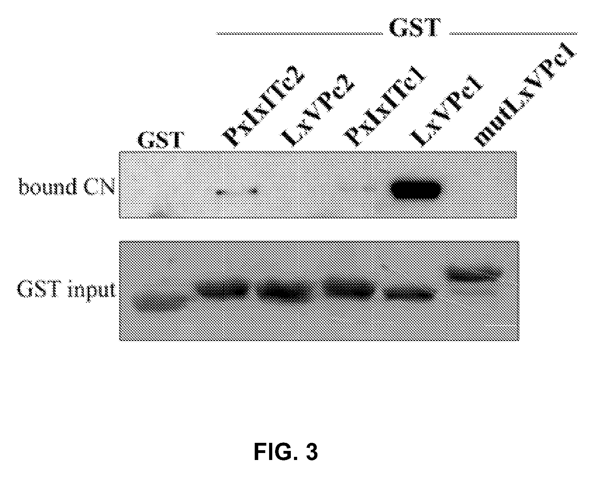

Figure 3:- Analysis of the *in vitro* interaction of CN with PxlxlT and LxVP peptides fused to GST. The GST-peptide fusion proteins containing the PxlxlT or LxVP sequences from NFATc1 and NFATc2 were used in pull-down tests. GST protein or GST fused to the mutated LxVPc1 (AxAA) peptide were used as negative controls. Bound CN was detected by Western blot and the quantity of GST fusion proteins used was analysed by means of staining total proteins on SDS-PAGE gels.

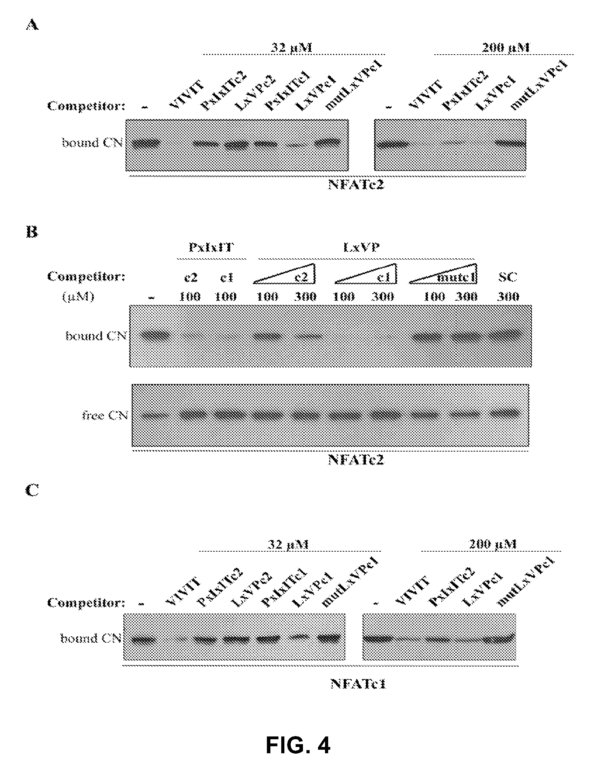

Figure 4- Effect of NFATc1 and NFATc2-derived PxlxlT and LxVP peptides on NFAT and CN *in vitro* interaction. GST-NFGAT-CN binding assays were performed in the presence of the indicated synthetic peptides, and bound CN was detected by Western blot with a monoclonal anti-CnA antibody. Figure 4A: CN bound to GST-NFATc2 in the presence of competitor peptides at 32 µM or 200 µM. The mutLxVPc1 peptide contains the LxVP motif from NFATc1 with the Leucine, Valine and Proline residues replaced by Alanine. Figure 4B: CN bound to GST-NFATc2 in the presence of high concentrations of competitor peptides. The SCRAMBLED control peptide (SC) was used as a negative control. The lower panel shows the unbound (free) CN in one third of the supernatants produced after the binding reaction. Figure 4C: CN bound to GST-NFATc1 in the presence of competitor peptides at 32 µM or 200 µM.

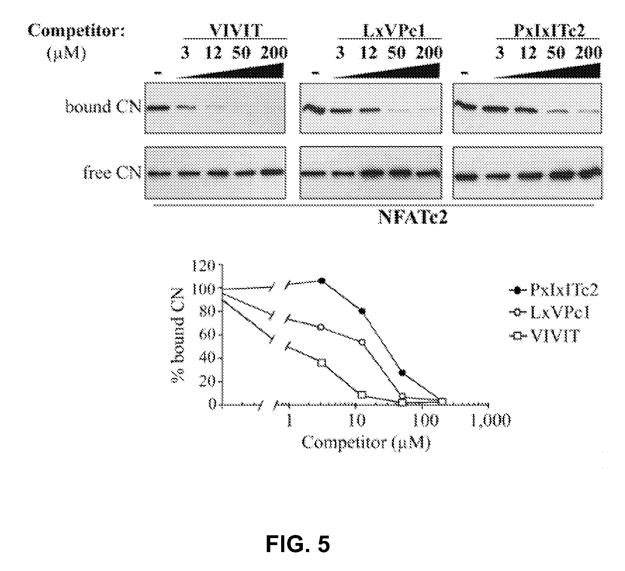

Figure 5:- Comparison of the capacities of VIVIT, LxVPc1 and PxIxITc2 peptides to block CN binding to NFATc2. GST-NFAT binding to CN was competed by increasing concentrations of VIVIT, LxVPc1 or PxIxITc2 peptides. The upper panels show the amount of CN bound to GST-NFATc2, while the lower panels show the unbound (free) CN in one third of the supernatants produced after the binding reaction. Bound CN was quantified by densitometric analysis with the *Quantity One* (BIO-RAD) software, and these data are presented in the graph to show the inhibition curves of each competitor peptide for the binding of CN to GST-NFATc2. 100% bound CN is assimilated to the amount of CN bound to GST-NFATc2 in the absence of competitor peptide.

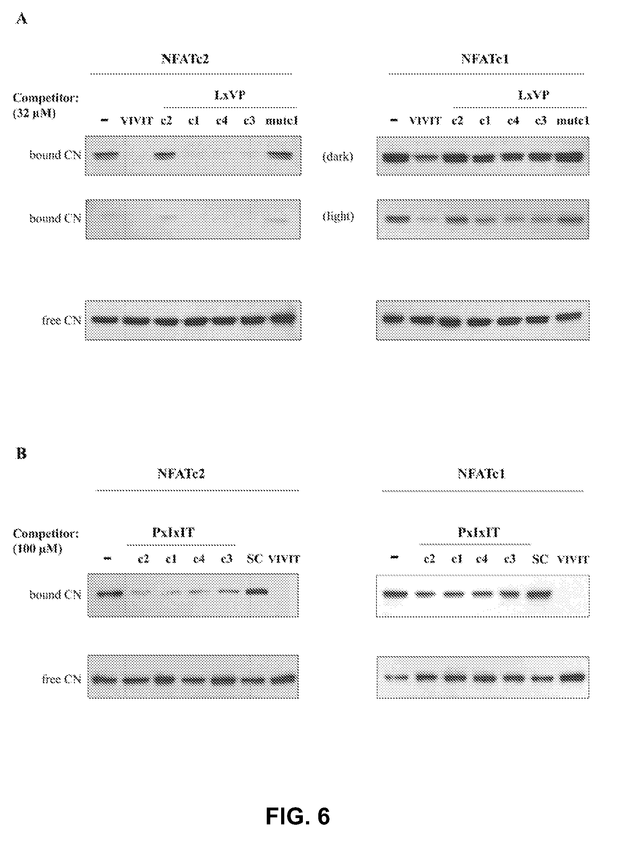

Figure 6:- Competition of the interaction of NFATc1-CN and NFATc2-CN by peptides spanning the PxlxlT and LxVP motifs of NFATc1, c2, c3 and c4. Figure 6A: Peptides corresponding to the LxVP motifs from NFATc1, c2, c3 and c4 (32 µM) were used to compete *in vitro* the binding of CN to GST-NFATc2 (left) or GST-NFATc1 (right). Because of the high amounts of CN bound to NFATc1, two different exposures are shown (top and middle panels) to show clearly the effect of the peptides on the NFATc1-CN interaction. The bottom panels show the unbound (free) CN in one third of the supernatants produced after the binding reaction. Figure 6B: *In vitro* GST-NFAT-CN binding in the presence of 100 µM of each PxlxlT peptide. Bound and unbound (free) CN are shown. The peptide sequences are shown in Figure 1.

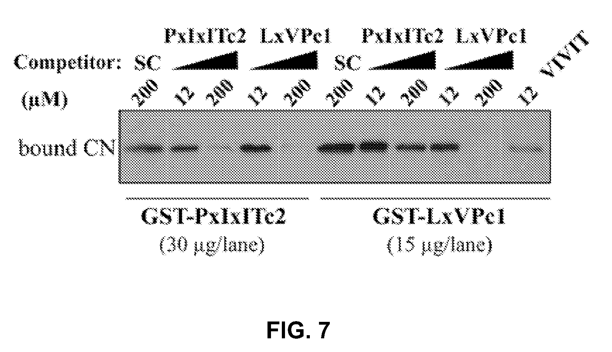

Figure 7:- Cross-competition of GST-PxlxlTc2-CN or GST- LxVPc1-CN interactions by PxlxlTc2 and LxVPc1 peptides. GST-peptide fusion proteins containing the PxlxlTc2 or LxVPc1 peptides were used in CN pull-down tests in the presence of free competitor peptides as indicated. The SCRAMBLED peptide (SC: 200 µM) was used as negative competitor control, and the effect of VIVIT peptide (12 µM) was also analysed. To better detect the scant amount of CN bound to GST-PxlxlTc2, the amount of GST-PxlxlTc2 used was double that of GST-LxVPc1.

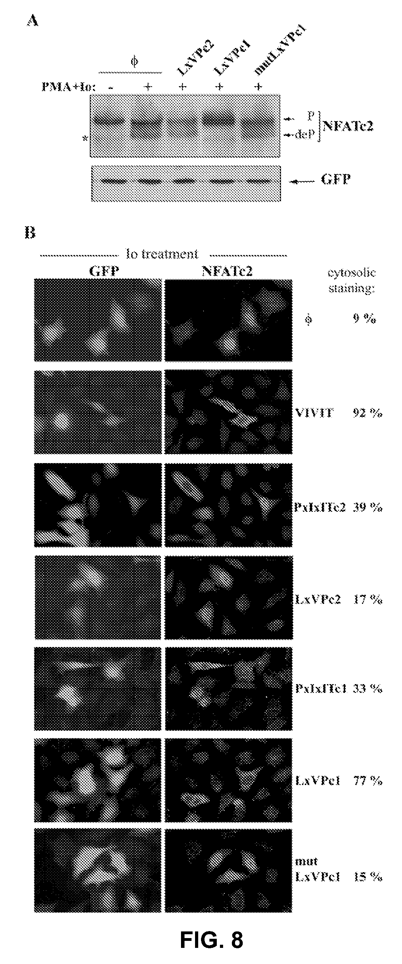

Figure 8:- Blockade of NFATc2 regulation by the LxVPc1 peptide *in vivo.* The HeLa (non expressing NFAT) cells were co-transfected with the HA-NFATc2 *wild type* constructs and the GFP fusion proteins with the PxlxlT or LxVP peptides derived from NFATc2 or NFATc1. The mutant LxVPc1 peptide (mutLxVPc1) fused to GFP was used as a control. Figure 8A: Transfected HeLa cells were stimulated -or not- with PMA (20 ng/ml) plus calcium ionophore (lo) (1 µM) (PMA+lo) for one hour. Total cell extracts were analysed by Western blot with either an anti-HA antibody for NFATc2 detection (upper panel) or an anti-GFP serum to control the expression of GFP fusion proteins (lower panel). The electrophoretic mobility of the upper (phosphorylated) and lower (dephosphorylated) bands corresponds to the state of NFATc2 phosphorylation. Extracts of cells expressing only the parental GFP protein are shown by the Φ lanes. The asterisks indicate non-specific bands. Figure 8B: Subcellular localisation of NFATc2 in transfected HeLa cells treated with lo for 30 minutes. The immunofluorescence images show representative fields of cells showing the corresponding GFP-peptide fusion protein (left) and the staining of NFATc2 (right). The percentage of cells with a cytosolic NFATc2 distribution is indicated (at least 130 cells per transfection were counted).

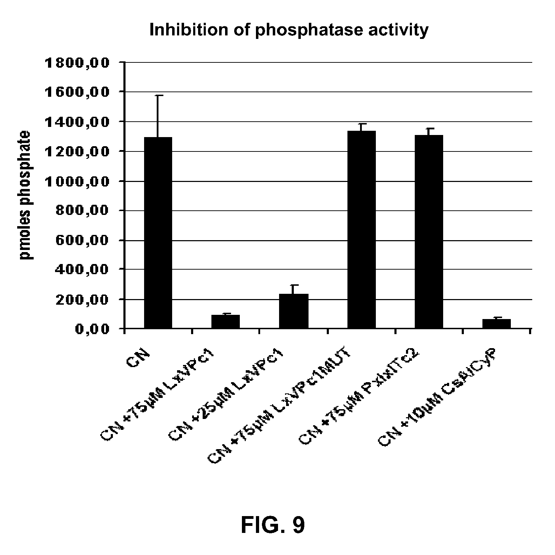

Figure 9:- The LxVPc1 peptide is a powerful inhibitor of CN phosphatase activity. The phosphatase activity of CN on the RII phosphopeptide substrate was measured in the absence and in the presence of different peptides (PxIxITc2, LxVPc1 and mutLxVPc1). The results were comparable to those obtained in the presence of 10 µM of the CsA/CyP inhibitory complex. The data are represented in picomoles of phosphates released during the reaction and are expressed as the mean +/- SD of an experiment, and were carried out in duplicate. One experiment, which is representative of the three assays performed, is shown.

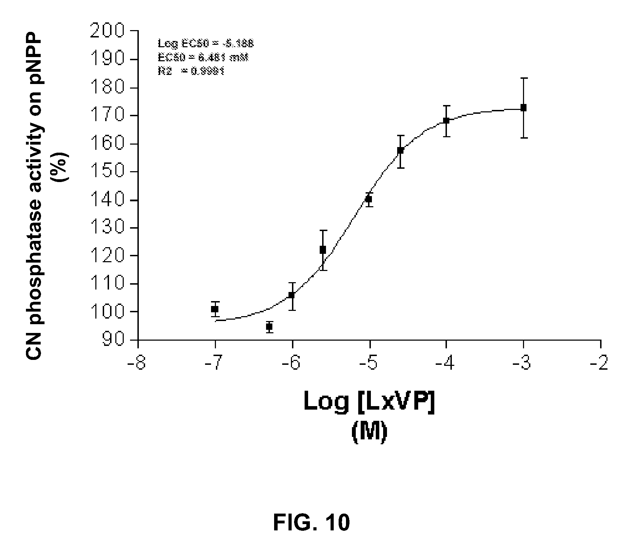

Figure 10:- The LxVP peptide increases the phosphatase activity of CN on pNPP (p-Nitrophenyl Phosphate) substrate. The capacity of hydrolysis of the pNPP phosphate group exercised by CN was analysed in the presence of increasing amounts of LxVPc1 peptide. This graph portrays the mean of three experiments and permits the calculation of the EC50 (Effective Concentration for 50% effect) of the peptide by CN, near to 6.5 mM.

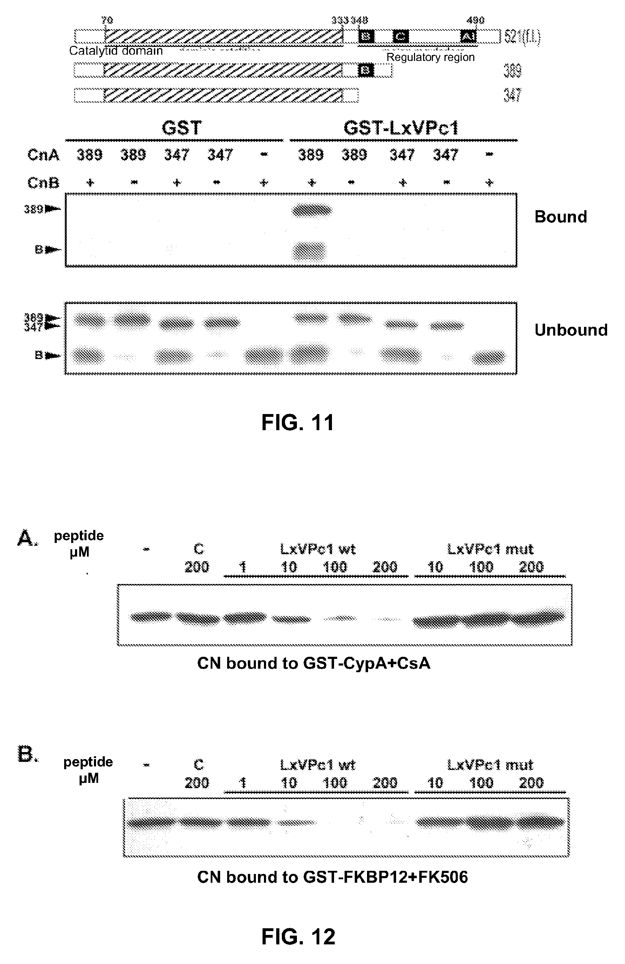

Figure 11:- the interaction of the peptide LxVP with CN requires the presence of the heterodimer. In this assay, the *in vitro* interaction of the sub-units CnA and CnB, separately or forming the heterodimer, with the GST protein fused to the sequence of the LxVPc1 peptide at its C-terminal end is compared. In the figure is shown that LxVPc1 only binds CN when the two sub-units, CnA plus CnB, are present. As a specifity control, the binding of the different sub-units of CN to the GST protein was analysed.
Figure 12:- the LxVPc1 peptide prevents interaction between CN and immunosuppressor drugs. The interaction assay was performed using GST-Cyclophilin-A (panel A) or GST-FKBP12 (panel B) in the presence of increasing amounts of wild or mutant LxVPc1 peptide. A control peptide which does not bind to CN was used as a control (C).

### DETAILED DESCRIPTION OF THE INVENTION

This invention is based on the fact that the inventors have discovered the surprising usefulness of LxVPc1, LxVPc3 and LxVPc4 peptides as efficient selective inhibitors of the CN-NFAT signalling pathway, and more particularly, as useful base immunosuppressor compounds for the preparation of therapeutic compositions for the prophylaxis and treatment of human diseases associated with the activation of T-lymphocytes such as, but not limited to, autoimmune diseases, inflammation and allergy or transplant rejection situations. Likewise, these peptides and the associated genetic and biological material may represent useful tools for the development of tests to find compounds which have selective antagonist activity against Calcineurin (hereinafter CN).

In this invention, the interaction of NFATc1 and NFATc2 with active CN has been compared. The semi-quantitative analyses clearly show that the interaction of CN with NFATc1 is stronger than with NFATc2. On the other hand, the competitive experiments using peptides based on the LxVP sites of the different NFATs suggest that NFATc3 and NFATc4 would have a CN-interaction capacity similar to that of NFATc1. Therefore, while the PxlxlT motif is the main docking site of CN in NFATc2, the presence of functional LxVP sites in NFATc1, NFATc3 and NFATc4 contributes to a stronger NFAT-CN interaction.

It has also been seen that the peptide LxVPc2 is a very weak competitor of the binding of CN to NFAT, which suggests that the NFATc2-CN interaction could be mainly due to the PxlxlT motif. The LxVP motif of NFATc1 presents different characteristics. This LxVP peptide binds to CN much more effectively than the PxlxlT motifs of NFATc1 and NFATc2 (Figure 3). It is also capable of inhibiting *in vitro* the interaction of CN with both NFATs and the translocation of NFATc2 in activated cells (Figures 4-6 and Figure 8). The great capability of NFATc1 of interfering in the NFATc2-CN complexes both *in vitro* and *in vivo* is one of the most surprising results of this invention. The LxVP peptides based on the NFATc1, NFATc3 and NFATc4 sequences have also proved to be efficient competitors of the *in vitro* interaction of CN with NFATc1 or NFATc2. It might therefore be expected that expression in cells of the peptides LxVPc3 and LxVPc4 would give rise to a similar effect to that observed with the LxVPc1 peptide on the activation of NFATc2.

On the other hand, in this invention, clear evidence appears that the residues conserved in the LxVP, Leucine, Valine and Proline motifs are essential for the interaction of NFATc1 with CN. However, the fact that the LxVPc2 motif also presents these amino acids conserved indicates that other residues present in said motif must be of importance in the protein-protein interaction. Beyond the three residues conserved, only NFATc1, NFATc3 and NFATc4 possess an aromatic amino acid adjacent to the nucleus of the LxVP motif (Tyrosine, Phenylalanine and Tyrosine, respectively). Another difference between the sequences is the presence in NFATc2 of a Proline residue adjacent to the C-terminal end of the nucleus of the LxVP motif, which gives rise to two consecutive Proline residues in which the remainder of the NFAT members are lacking. This makes it possible for the differences in the regions which flank the core of the motif to explain the differential behaviour of LxVPc2 compared with LxVPc1, LxVPc3 and LxVPc4.

Another surprising result of this invention is that the LxVPc1 peptide binds directly to CN and inhibits its phosphatase activity at levels similar to CsA (see Figure 9, example 7). Thus, the use of this peptide LxVPc1 as an immunosuppressor would offer clear advantages over the immunosuppressant agents CsA and FK506. Administration of the peptide LxVPc1 would inhibit the phosphatase activity of CN, without affecting other non-CN-dependent cell processes and which could be responsible for some of the side-effects associated with treatment with CsA and FK506 drugs (31-33); requiring the dimeric structure (CnA+CnB) to bind to CN. Furthermore, treatment with this peptide would be suitable for distinguishing between the side-effects due to the inhibition of the CN phosphatase activity and those which are independent of CN.

Therefore, a first aspect of this invention refers to a sequence of amino acids, capable of selectively inhibiting the CN-NFAT signalling pathway comprising the amino acid (aa) sequence:

R₁-L-R₂-V-P-R₃

wherein R₁ is an aromatic aa of the Tyrosine or Phenylalanine type, R₂ is an Alanine or Serine-type amino acid and R₃ is not the amino acid Proline (hereinafter we shall refer to this amino acid sequence as the basic starting nucleus of the invention).

Said amino acid sequence forms the basic starting core for the setting-up of all those compounds capable of selectively inhibiting the CN-NFAT signalling pathway, and more specifically, capable of inhibiting the biological action of CN on, at least, the transcription factors NFATc1 and NFATc2.

Another preferred embodiment of this invention consists of a sequence of amino acids, capable of selectively inhibiting the CN-NFAT signalling pathway and more particularly, capable of inhibiting the biological action of CN on the NFATc1 and NFATc2 transcription factors, possessed by the basic starting nucleus of the invention and which belongs to the following group:
a) aa sequence SEQ ID NO 18 (LxVPc1) or SEQ ID NO 21 (LxVPc3) or SEQ ID NO 22 (LxVPc4),
b) aa sequence analogous to the sequence in (a)
c) aa sequence comprising any sequence belonging to (a) or (b).

In the sense used in this description, the term "analogous" is intended to include any aa sequence which may be isolated or constructed on the basis of the aa sequences shown in this invention, for example, by means of the insertion of aa substitutions, conservative or non-conservative, including the insertion of one or more amino acids, the addition of one or more aa, at any of the ends of the molecule, or the deletion of one or more aa, at any end or in the interior of the sequence, on condition that this shall not modify the basic nucleus of the invention and that it shall constitute a peptide capable of selectively inhibiting the CN-NFAT signalling pathway, and more specifically, capable of inhibiting the biological action of CN on the transcription factors NFATc1 and NFATc2.

In general, an analogous aa sequence is fundamentally homologous to the aa sequence mentioned above. In the sense used in this description, the expression "fundamentally homologous" means that the aa sequences in question shall have a degree of identity of at least 40%, preferably of at least 85%, or more preferably still, of at least 95%.

A particular aspect of the invention is constituted by a peptide capable of selectively inhibiting the CN-NFAT signalling pathway, and more specifically, capable of inhibiting the biological action of Calcineurin on the transcription factors NFATc1 and NFATc2, and which comprises the amino acid sequence SEQ ID NO 18 or an aa sequence analogous or fundamentally analogous to SEQ ID NO 18.

Another particular aspect of the invention is constituted by a peptide capable of selectively inhibiting the CN-NFAT signalling pathway, and more specifically, capable of inhibiting the biological action of Calcineurin on the transcription factors NFATc1 and NFATc2, and which comprises the amino acid sequence SEQ ID NO 21 or an aa sequence analogous or fundamentally analogous to SEQ ID NO 21.

Yet another particular aspect of the invention is constituted by a peptide capable of selectively inhibiting the CN-NFAT signalling pathway, and more specifically, capable of inhibiting the biological action of CN on the transcription factors NFATc1 and NFATc2, and which comprises the amino acid sequence SEQ ID NO 22 or an aa sequence analogous or fundamentally analogous to SEQ ID NO 22.

Any of the aa sequences of this invention may be produced by the gene expression of the nucleotide sequences which permit the encoding of their residues, likewise by means of their chemical synthesis. Some modifications may be of importance in order to establish the peptide in future pharmaceutical formulations in which it may be included for administration to a person or for its use as a research tool. Likewise, one or more nucleotides of the codon corresponding to each residue of interest may be replaced.

Therefore, the aa sequences of the peptides of this invention and the nucleotide sequences which encode the same are comprised of a series of sequences which any person skilled in the art may produce in a routine manner, without excessive experimentation, from the information provided in this invention. Said replacements or modifications, suitable for carrying out the various aspects of this invention may be determined by means of routine experimentation in order to produce peptides with the structural and functional properties described, for example by comparing the protein-protein interactions by means of *in vitro* or *in vivo* competition assays using CN with any one of the NFATs c1-c4.

Any combination of deletion, insertion and replacement may be carried out in order to express and produce the final construct of the desired peptide by means of mutations in the DNA and genetic engineering techniques using host cells which include said nucleotide sequences.

Therefore, another aspect of this invention includes a nucleotide sequence capable of encoding the aa sequence of the LxVPc1, LxVPc3 or LxVPc4 peptides of this invention comprising a nucleotide sequence selected from one of the following groups:
a) the nucleotide sequence SEQ ID NO 5 and/or its complementary sequence SEQ ID NO 6.
b) A nucleotide sequence and/or its complementary encoding sequence for the amino acid sequence SEQ ID NO 21.
c) A nucleotide sequence and/or its complementary encoding sequence for the amino acid sequence SEQ ID NO 22.
d) A nucleotide sequence analogous to sequences (a), or (b) or (c).
e) A nucleotide sequence which comprises any one sequence belonging to (a), (b), (c) and (d).

A particular aspect of this invention is comprises a nucleotide sequence capable of encoding an amino acid or peptide sequence capable of selectively inhibiting the CN-NFAT signalling pathway, and more particularly, of inhibiting the biological action of CN on the transcription factors NFATc1 and NFATc2, and which comprises the nucleotide sequence SEQ ID NO 5 and/or its complementary sequence SEQ ID NO 6.

The nucleotide sequences described in this invention may also be bound or fused, if necessary and in order to allow better isolation or detection of the peptide expressed, to an encoding DNA sequence for a peptide which may be used for the purpose of isolation or detection of said peptide.

Therefore, another particular aspect of this invention consists of a genetic construct which is also comprised of a nucleotide sequence selected from among the SEQ ID NO 5 nucleotide sequence and/or its complementary sequence SEQ ID NO 6, or the encoding nucleotide sequence for the LxVPc3 peptide, or the encoding nucleotide sequence for the LxVPc4 peptide, any other peptide encoding nucleotide sequence or peptidic sequence which permits the isolation or detection of the peptide(s) mentioned.

The nucleotide sequence and the genetic construct of this invention may be produced by means of the use of techniques which are commonly known by any person skilled in the art (34). Said nucleotide sequences may be integrated into an expression vector which permits regulation of the same in conditions which are suitable for the production of the corresponding peptides or proteins which are an active pharmaceutical ingredient.

Therefore, yet another particular aspect of this invention consists of a gene expression vector which is comprised of the LxVPc1 nucleotide sequence and/or its complementary sequence or the LxVPc3 nucleotide sequence and/or its complementary sequence or the LxVPc4 nucleotide sequence and/or its complementary sequence or the LxVPc1 genetic construct or the LxVPc3 genetic construct or the LxVPc4 genetic construct of this invention and which permits the expression of said construct in the cytoplasm of a cell of the invention.

In general, the LxVPc1 expression vector or the LxVPc3 expression vector or the LxVPc4 expression vector of this invention comprises, in addition to the LxVPc1 nucleotide sequence and/or its complementary sequence or the LxVPc3 nucleotide sequence and/or its complementary sequence or the LxVPc4 nucleotide sequence and/or its complementary sequence or the LxVPc1 genetic construct or the LxVPc3 genetic construct or the LxVPc4 genetic construct of this invention, a promotor which directs its transcription, to which it is operatively linked, and other necessary or appropriate sequences which monitor and regulate said transcription and, where applicable, the translation of the product of interest, for example transcription start and stop signals, polyadenylation signals, replication origin, ribosome binding sequences, transcriptional regulator encoding sequences, transcriptional silencers, repressors, etc. Examples of suitable expression vectors may be selected according to the conditions and needs of each particular use among cell expression plasmids which may also contain markers which may be used for selecting the cells transfected or transformed with the gene or genes of interest. The choice of vector will depend on the host cell and on the type of use desired.

Therefore, in accordance with a particular embodiment of this invention, said vector is a plasmid or a viral vector. The production of said vector may be carried out by means of conventional methods by those skilled in the art as, for the transformation of micro-organisms and eukaryote cells different methods may be used, such as, but not limited to, chemical transformation, electroporation, micro-injection, among others. One strategy could be that of using lentivirus in order to infect the target cells, as is already being attempted in gene therapy by Ralph et al. (35).

These aforementioned nucleotide sequences may be used in a gene therapy process in which by means of any technique or procedure, the integration of the same into the cells of a human patient may be achieved. This goal may be achieved by means of administering to these cells a gene construct comprised of one or several of these aforementioned nucleotide sequences in order to transform said cells permitting their expression in the interior of the same in order to inhibit the CN-NFAT signalling pathway. Conveniently, said gene construct may be included within a vector such as, for example, an expression vector or transference vector.

In this way, the nucleotide sequence, the gene construct or the expression vector may be used as a drug in order to protect human host cells in a procedure of treatment and prophylaxis of *in vivo* or *ex vivo* gene therapy of a human being affected by a disease entailing exacerbation of the immune response. The gene therapy procedures may be applied directly on the patient, administering one of these aforementioned nucleotide sequences as a drug, or exacerbated immune system cells could be extracted from a human being, and once *ex vivo,* they could be transformed with the nucleotide sequences and subsequently returned to the human being, or maintained *ex vivo* for subsequent uses.

Biopharmaceutical tools and gene therapy procedures are sufficiently well-known by those skilled in the art so that with the information described in this invention they may be developed with no excessive effort.

An additional aspect of this invention consists of a transformed cell which comprises a sequence of LxVPc1 nucleotides and/or their complementary sequence or a sequence of LxVPc3 nucleotides and/or their complementary sequence or a sequence of LxVPc4 nucleotides and/or their complementary sequence or an LxVPc1 genetic construct or an LxVPc3 genetic construct or an LxVPc4 genetic construct or an LxVPc1 vector or an LxVPc3 vector or an LxVPc4 vector of this invention and which is capable of expressing the LxVPc1 peptide or the LxVPc3 peptide or the LxVPc4 peptide respectively of this invention under suitable conditions. The LxVPc1 cell or the LxVPc3 cell or the LxVPc4 cell of the invention may be a prokaryote or eukaryote cell in accordance with particular needs and may be produced by a person skilled in the art with the information disclosed in this invention.

These cells may be useful for the production of the peptides with CN-NFAT signalling pathway inhibitory activity, which may be the basis of a pharmaceutical composition for the recombinant amplification of said nucleotide sequences or may be useful *per se* as cells in gene therapy.

On the other hand, the nucleotide sequence, genetic construct, expression vector, the transformed cells and the LxVPc1, c3 or c4 peptides of this invention may be used directly, or by means of their genetic expression, in host cells for the production of identification or screening systems for CN-NFAT signalling pathway regulatory pharmaceutical compounds, more specifically that of the biological action of CN on the transcription factors NFATc1 and NFATc2, preferably selective antagonists of CN-NFAT by means of the determination of the effect of the addition of a potential compound on said activity. In these assays different combinations may be used, one or more of the possible potential forms of the LxVPc1, c3 or c4 peptides. Direct use of the LxVPc1, c3 or c4 peptide of this invention may be used, after extraction and purification, in an *in vitro* assay of protein interaction or binding. In these assays, the proteins or fragments of the same involved in the interaction for which it is desired to identify regulatory compounds may be used; while the interaction of said proteins may be assessed for example by means of Western blot (see examples). The compounds to be identified may be of various origins: natural (plants, prokaryote, etc) or synthetic. This screening system can measure or detect (quantitatively or qualitatively) the competitive binding of a candidate compound to a signal peptide by means of the direct or indirect marking of one of the elements of said system.

Therefore, another aspect of this invention comprises a procedure or assay for the identification or screening of new regulatory pharmaceutical compounds, preferably selective antagonists, of the CN-NFAT signalling pathway, more specifically that of the biological action of CN on the transcription factors NFATc1 and NFATc2 and which comprises the use of the nucleotide sequence, gene construct, expression vector, the host cells and/or the peptides of this invention, in an isolated manner or in one of their possible combinations. Another aspect of this invention comprises a pharmaceutical compound identification assay in which the procedure is a pharmaceutical compound identification assay in which the procedure is an *in vitro* assay or an *in vivo* assay such as, for example, a co-immunoprecipitation or an immunodetection assay.

As used in this invention, the term "selective" relates to the fact that the capability of the peptide of this invention of acting as an antagonist of CN activity is considerably greater than its capability of acting as an antagonist of other proteins and, on the other hand, that more specific peptides may be selected in order to inhibit different activities of the same CN protein.

Therefore, another aspect of this invention comprises a pharmaceutical composition which is useful for the treatment of diseases, hereinafter the pharmaceutical composition of the invention, together with, optionally, one or more adjuvants and/or pharmaceutically acceptable vehicles, comprising any of the aforementioned peptides (SEQs ID NO 18, 21 and 22) including their analogues and substantially analogues or any other compound comprising the basic nucleus, or comprising any of the aforementioned nucleotide sequences, gene constructs or expression vectors or their blends, they being capable of selectively inhibiting the CN-NFAT signalling pathway associated with CN activation, and preferably, for illustrative purposes and without limiting the scope of the invention, with activation of the immunological system.

The adjuvants and pharmaceutically acceptable vehicles which may be used in said compositions are the adjuvants and vehicles known by those skilled in the art and habitually used in the preparation of therapeutic compositions.

The pharmaceutical composition provided by this invention may be administered by any suitable administration route; to this end, said composition shall be formulated in the pharmaceutical format suited to the administration route chosen.

The pharmaceutical composition of this invention may be used in a method of treatment in an isolated manner or jointly with other pharmaceutical compounds.

Another aspect of this invention comprises the use of the pharmaceutical composition of the invention in the prophylaxis and treatment of human diseases associated with CN activation, and preferably, for illustrative purposes and without limiting the scope of the invention, with T-lymphocyte activation such as, for example, auto-immune diseases, inflammation, allergy or transplant rejection situations.

Finally, another aspect of this invention comprises the use of the pharmaceutical composition of the invention as a tool for basic research for the isolation of new CN substrates which are important for calcium-mediated signalling in different species.

The following examples and figures are for illustrative purposes but do not limit this invention.

### EXAMPLES OF EMBODIMENTS

### Example 1:- CN binding to NFATc1 is stronger than to NFATc2

In order to analyse the interaction capacity of NFATc1 and NFATc2 with CN, several binding assays were carried out *in vitro.* CN bound to decreasing concentrations of the purified recombinant regulatory domains of NFATc1 or NFATc2 fused to the protein GST (GST-NFATc1 and GST-NFATc2 respectively) were compared. Quantification by densitometry of the bound CN showed that NFATc1 has a binding capacity to CN of a submicromolar nature, and that the affinity of NFATc2 appears to be much lower than that of NFATc1 (Figure 2A)

Complementary experiments were carried out in which the concentration of CN was varied. In each case, the quantity of CN bound to the GST-NFAT fusion proteins decreased as the concentration of CN was progressively reduced. However, and in accordance with the results obtained when varying the concentrations of GST-NFAT, at each concentration of CN assayed, the bind to NFATc1 was stronger than to NFATc2 (Figure 2B).

### Example 2:- CN binding capacity to the LxVPc1 motif is greater than to the PxlxlT motifs.

These differences in CN binding might be due to a differential CN binding capability to the PxlxlT and LxVP sites of NFATc1 and NFATc2. The second CN binding sites show a limited homology between the different NFAT members, although their alignment indicates that there are three conserved residues in all of the members (Leucine, Valine and Proline) (Figure 1A). In order to test the interaction capability of each independent PxlxlT and LxVP sequence with CN, we fused the GST protein with the PxlxlT and LxVP motifs of NFATc1 and NFATc2 (Figure 1A), and we used them in pull-down experiments with CN (Figure 3). The amount of CN bound to GST-LxVPc1 was much greater than that bound to GST-PxlxlTc1 or GST-PxlxlTc2, while GST-LxVPc2 was incapable of binding CN under the same experimental conditions. The relevance of the amino acids conserved (Valine, Leucine and Proline) for the binding of LxVPc1 to CN was studied using a GST-mutLxVPc1 construct, in which each conserved residue was replaced by Alanine; GST-mutLxVPc1 was incapable of binding CN (Figure 3). This result suggests that the greater binding capability of CN to the LxVP motif of NFATc1 (with regard to the PxlxlTc1 and PxlxlTc2 motifs) could be responsible for the greater strength in CN interaction with NFATc1 versus NFATc2.

### Example 3:- Effect of the PxlxlT and LxVP peptides on NFAT-CN interaction in vitro.

Given the differences in the interaction capability of CN with the PxlxlT and LxVP sites, the capability of the peptides corresponding to said sequences of competing the binding of GST-NFATc1 with CN *in vitro* was assessed. The synthetic peptides corresponding to the CN LxVP and PxlxlT binding sequences of NFATc1 and NFATc2 are shown in Figure 1A. The amount of CN bound to GST NFAT was detected by Western blot analysis. In these assays, the VIVIT peptide (Figure 1 B), a highly specific sequence selected by means of a combinatorial peptide library (29), was used as a potent control competitor.

The PxlxlT peptides corresponding to NFATc1 and NFATc2 (PxlxlTc1 and PxlxlTc2, respectively) had a similar potency displacing the binding of NFATc2 and CN (Figure 4A), but there were clear differences between the two peptides based on the LxVP sequences. LxVPc1, the peptide which contained the LxVP sequence of NFATc1, interfered with the binding of CN to NFATc2. This effect was even stronger than that caused by the PxlxlTc2 sequence, described as the CN docking site to this NFAT (Figure 4A). Conversely, the same concentration of the NFATc2-specific LxVP peptide (LxVPc2) was incapable of competing the binding of NFATc2 to CN (Figure 4A, left panel). In these assays, the mutLxVPc1 peptide did not affect the binding of NFATc2 to CN, indicating that these residues are necessary in order to compete the binding of NFAT to CN *in vitro.*

These results suggest that LxVPc2 does not interact with CN, or at least in depth, that its interaction capability is much weaker than that of LxVPc1. In order to look at this point in greater detail, we increased the concentration of competitor peptides in the binding reactions. As is portrayed in Figure 4B, while a concentration of 100 µM of PxlxlTc1, PxlxlTc2 or LxVPc1 efficiently blocked the interaction of CN with NFATc2, LxVPc2 was incapable of affecting the binding at this concentration, and using concentrations as high as 300 µM only brought about a partial inhibition. On the other hand, both the control peptide mutLxVPc1 and another peptide which contained a random distribution of the amino acids of the VIVIT (scrambled) (see Figure 1B) were incapable of affecting the binding of CN to NFATc2, even at the highest peptide concentrations.

The peptides which prevented the CN-NFATc2 interaction were also capable of inhibiting the binding of NFATc1 to CN (Figure 4C). Consistently with the differential binding capability of CN to NFATc1 and NFATc2 observed (Figure 2), the peptides, even at 200 µM, were less effective competing the binding of CN with NFATc1 than with NFATc2 (Figure 4C). These results further reinforce the hypothesis that the global affinity of NFATc1 to CN is greater than that of NFATc2.

### Example 4:- The LxVPc1 peptide is a potent inhibitor of the binding of CN and NFAT.

"Dose-dependent" competition experiments confirmed that the peptide LxVPc1 is more potent than LxVPc2 competing the GST-NFATc2 binding to CN (Figure 5). In these experiments, the amount of bound CN was analysed by computerised quantification of Western blot assays. The VIVIT peptide, used as a positive control, was the most potent competitor (Figure 5, left panel). A concentration of 50 µM of the LxVPc1 peptide totally blocked the binding, while the same concentration of PxIxITc2 produced only a partial inhibiting effect (Figure 5, central and right panels).

The differences between the inhibitory capability of the LxVPc1 and LxVPc2 peptides caused the study to be extended to the LxVP motifs of the remaining members of NFAT. The synthetic peptides corresponding to the LxVP sequences of NFATc1, c3 and c4 inhibited the interaction of GST-NFATc1 with CN in a similar way when they were used at 32 µM (Figure 6A); only the LxVPc2 and mutLxVPc1 (used as a negative control) peptides were not capable of altering the NFAT-CN binding at that concentration. In parallel experiments, we used the peptides based on the PxlxlT sequences of all the NFAT members as competitors. As may be seen in Figure 6B, a dose of 100 µM of all the PxlxlT peptides (three times greater than the concentration used for the LxVP peptides) was capable of blocking the interaction of CN with NFATc1 or NFATc2, while the scrambled control peptide was not capable of doing so. Of all the peptides corresponding to the two docking sequences of the different NFATs, only LxVPc2 did not efficiently compete the *in vitro* interaction of CN with NFATc1 or NFATc2; the remaining LxVP peptides have proved to be better competitors than the PxlxlT peptides.

### Example 5:- Cross-competition between the different sequences of binding the NFATs to CN.

The capacity possessed by the LxVPc1 peptide for competing the binding of CN to NFATc2 (Figures 4, 5 and 6) was surprising. In order to go into this fact in greater detail, new competition experiments were performed, this time to analyse the capability of the peptides to compete the binding of CN to some GST-peptide fusion proteins. The LxVPc1 peptide competed the binding of CN to GST-PxlxlTc2; likewise the PxlxlTc2 and VIVIT (peptide derived from the PxlxlT sequence) peptides competed the binding of CN to GST-LxVPc1 (Figure 7). Given the lack of clear similarities between these two NFAT motifs, the result suggests that the CN sequences which interact with the PxlxlT and LxVP motifs of NFAT must be in some way interdependent.

### Example 6:- The in vivo expression of the LxVPc1 peptide blocks NFATc2 activation.

In order to analyse whether the peptides derived from the binding sites of CN to NFATc1 had the capability of regulating NFATc2 in living cells, a *wild type* NFATc2 expression vector was co-transfected with another which encoded the GFP-LxVP or PxlxlT fusion proteins of NFATc1 and NFATc2. The HeLa cell line was used because it lacks endogenous NFAT expression. As positive and negative controls, the GFP-VIVIT or GFP-mutLxVPc1 fusion proteins were used, respectively.

Analysis of total cell extracts by means of Western blot showed that GFP-LxVPc1 expression blocked induced NFATc2 dephosphorylation by increasing intracellular calcium (Figure 8A); on the other hand, the expression of GFP, GFP-LxVPc2 or GFT-mutLxVPc1 had no effect. However, and in apparent discrepancy with data previously published (15), no significant effect on the dephosphorylation of NFATc2 was detected when the GFP-PxlxlT constructs were expressed (data not shown). Given that the cause of this discrepancy could be the presence of NFATc2 in negative GFP cells, the direct effect of the GFP-peptide constructs on the nuclear translocation of NFATc2 was analysed by immunofluorescence. Analysis of the control immunofluorescences of non-stimulated cells showed that NFATc2 was to be found in the cytoplasm of the cells transfected with the different GFP constructs (data not shown). In the majority of the cells transfected with the GFP-peptide construct control, NFATc2 was localised in the nucleus after activation with calcium ionophore, only 9% of the cells showing cytosolic NFATc2 staining (Figure 8B). As with the dephosphorylation of NFATc2, the greatest inhibiting effect on the nuclear translocation of NFATc2 was achieved with the expression of GFP-VIVIT and GFP-LxVPc1: 92% and 77% of cells with cytosolic NFATc2 staining, respectively. Besides, it was observed that GFP-PxlxlTc2 or GFP-PxlxlTc1 expression partially inhibited the nuclear translocation of NFATc2: between 33% and 39% of positive GFP cells showed cytosolic localisation of NFATc2 after activation (Figure 7B), data which agree with those published by Aramburu *et al.* (15). These results also coincided with the *in vitro* binding studies, and clearly indicated that the LxVPc1 peptide retained its capability of interacting strongly with CN and of inhibiting its *in vivo* interaction with NFATc2, preventing the nuclear translocation and dephosphorylation of NFATc2.

### Example 7:- The LxVPc1 peptide is an inhibitor of the phosphatase activity of CN on one of its physiological substrates, the RII peptide.

The results observed in Figure 6C show that LxVPc1 is more effective as an inhibitor of NFAT/CN interaction in the presence of CaM. Under these conditions, the CN self-inhibition domain would be beyond the active centre. Therefore, the question arose as to whether the LxVPc1 peptide could have an influence on the phosphatase activity of CN. In order to analyse this, the phosphatase activity of calcineurin was measured *in vitro* in the presence of different peptides. In accordance with previous results, it was found that the PxlxlT peptide failed in the inhibition of phosphatase activity (Figure 9). However and markedly so, the addition of the LxVPc1 peptide (but not its mutant version) severely blocked *in vitro* the phosphatase activity of CN on its physiological substrate: the phosphorylated peptide of the regulatory sub-unit II of the cAMP-dependent kinase protein (RII peptide). This new discovery suggests that the LxVPc1 peptide could bind near the active centre of CN or that the interaction of LxVPc1 with CN could affect the active conformation of CN.

### Example 8:- The LxVPc1 peptide is an activator of the phosphatase activity of CN on pNPP (para-nitrophenylphosphate).

As is the case with the immunosuppressor drugs CsA and FK506, the LxVPc1 peptide blocks the phosphatase activity of CN on the RII peptide (Figure 9) (24). Due to this similitude in its action, the question arose as to whether the LxVPc1 peptide would behave similarly to the drugs when its effect on the phosphatase activity of CN was analysed, using pNPP as a substrate. This small molecule binds directly to its active CN site. Regarding the complex immunodepressants CsA/CyPA and FK506/FKBP12 it has been described that their presence increases the phosphatase activity of the enzyme on pNPP (36, 24). This contrasting effect of the complex drugs/immunophilins on CN activity on pNPP which suggested that the drugs inhibited non-competitively the phosphatase activity without affecting the active centre, was subsequently proved by means of an exhaustive enzymatic analysis (37). As shown in Figure 10, the addition of the LxVPc1 peptide also has an activating effect on the CN-mediated dephosphorylation of pNPP. Table I shows both the activation of CN mediated by increasing amounts of the LxVPc1 peptide, and the increase in phosphatase activity induced by the CsA/CyPA complex. In Table I, the effect caused by the LxVPc1 peptide is compared with that produced in the presence of the CsA/CyPA complex, which has been described as increasing the dephosphorylation of pNPP by CN. The mutant LxVPc1 peptide, CsA and cyclophilin A were used separately as negative controls.
**Table I**.- Effect on the phosphatase activity of CN on pNPP in the presence of different peptides.

**Table I**

| **peptide** | **% CN activity** |
|---|---|
| **none** | **100.0 ± 0.0** |
| **0.1 mM LxVP** | **168.0 ± 9.6** |
| **1.0 mM LxVP** | **172.7 ± 18.6** |
| **1.0 mM LxVP mut** | **94.3 ± 3.5** |
| **0.01 mM CsA** | **106.3 ± 7.5** |
| **0.01 mM CypA** | **103.3 ± 7.1** |
| **0.01 mM CsA + CypA** | **197.3 ± 18.0** |

### Example 9:- The LxVPc1 peptide requires the dimeric structure (CnA+CnB) in order to bind to the CN phosphatase.

Crystallographic analysis of the CsA/CyPA and FK506/FKBP12 complexes bound to CN revealed that the immunosuppressor complexes interact with amino acids from both sub-units of the CN heterodimer, the catalytic sub-unit (CnA) and the regulatory sub-unit (CnB) (38). In order to analyse the structure requirements of the interaction of the LxVPc1 peptide with CN, *in vitro* binding assays were performed. To do this, different truncated CnA proteins (Figure 11) and also CnB sub-unit were separately expressed in bacteria, subsequently purified and used in these assays. CnA bound to the LxVPc1 peptide fused to the GST protein (GST-LxVPc1) was compared in the presence or absence of CnB. The protein CnA 347 which does not present the CnB binding domain was incapable of binding to GST-LxVPc1 (Figure 11). However, the protein CnA 389, which maintains the CnB binding domain, interacts specifically with GST-LxVPc1 only when the sub-unit CnB is added to the assay. These results clearly show that the interaction of the LxVPc1 peptide with phosphatase requires the heterodimeric CN structure, formed by the CnA and CnB sub-units.

### Example 10:- The LxVPc1 peptide blocks interaction between CN and immunosuppressor drugs.

As has been seen above, the LxVPc1 peptide shows a behaviour which is similar to that described for the CsA/CyPA and FK506/FKBP12 complexes with regard to its action on CN. In order to confirm these data it was decided to find out whether the LxVPc1 peptide was capable of displacing the *in vitro* interaction of CN with the drug/immunophilin complexes. In the presence of increasing concentrations of the LxVPc1 peptide, and not of its mutant version (mutLxVPc1), the amount of CN which interacts with the drugs bound to GST-CyPA or GST-FKBP12 drops progressively (Figure 12). These results indicated that the LxVPc1 peptide competes with the drug/immunophilin complexes by means of its interaction with CN.

### MATERIALS AND METHODS

### Gene constructs

The GST-NFATc1 constructs expressed by the regulatory region of NFATc1 (1-418 aa) fused to the *glutathione S-transferase* (GST) protein was kindly provided by Dr A. Rao (12). The GST-NFATc2 construct was generated by means of phased subcloning, starting from a cDNA which encoded the sequence from NFATc2 amino acid 4 to 385 on a pGEX4T3 vector (Amersham Biosciences) digested with *BamH1-Smal:*
The GST-peptide and GFP-peptide expression vectors were generated by means of phased cloning on the digested pGEX (Amersham Biosciences) or pEGFP-C (Clontech) vector of ring oligonucleotides with protruding ends which encoded the different peptidic sequences.
The expression plasmid pEF-BOS HA-NFATc2 has been described above (39), and it encodes the complete NFATc2 protein fused to the HA (*influenza virus hemagglutinin*) epitope at its NH₂-terminal end.
The construct which expresses CN (CnA in tandem with CnB) fused to GST, for its subsequent purification and use in the assays on anti-pNPP enzymatic activity, was generated from a prior plasmid described above (40), which expresses CN fused to a 6-histidine epitope.
The constructs which express the immunophilins fused to GST were created from constructs described (FKBP12 human: 41; Cyclophilin A: 42).
The constructs which express CnA and CnB fused separately to GST were generated by using PCR products amplified from total RNA of eukaryote cells. The different deletions of CnA were created by mutating the DNA sequence which encodes a termination codon for the amino acid of the position indicated by means of directed mutagenesis using Stratagene's "Quikchange mutagenesis kit", following the manufacturer's instructions.

### Transfection and analysis by Western blot of cell extracts.

The HeLa cells were cultivated in Dulbecco medium modified by Eagle (DMEM) (Gibco) completed with 10% bovine foetal serum (FBS) (Sigma). The day prior to transfection, the cells were seeded in 6-well plates in order to reach an 80% confluence at the moment of transfection. The cells were transfected with 18 ng of pEF-BOS HA-NFATc2 plus 1.1 µg of the GFP-peptide plasmids with 4 µl of Plus reagent plus 6 µl of Lipofectamine reagent (Invitrogen) for 3 hours in 1 ml of OPTIMEM medium (Gibco). By means of flow cytometry, the transfection efficiency and the percentage of cells which expressed GFP were quantified. Similar transfection efficiencies were obtained for the different constructs and in independent experiments.

Twenty-four hours after the transfection, the HeLa culture medium was replaced by DMEM+1%FBS. After a further two hours, the cells were stimulated during 1 hour using 20ng/ml of PMA (*phorbol 12 myristrate 13 acetate*) (Sigma) plus 1 µM of calcium ionophore A23187 (Sigma) and 3 mM of CaCl₂. Subsequently, the cells were washed using a cold saline phosphate buffer (PBS) and the cell extracts were obtained using a breaking buffer [20 mM Hepes pH 7.6 with 0.4 M of NaCl, 1 mM EDTA, 3 mM EGTA, 1 µM DTT (*dithiothreitol*), 1mM PMSF (*phenylmethylsulfonyl fluoride*), 100 µM benzamidine, 1 µg/ml of pepstatine, 1 µg/ml of aprotinine and 1% of Triton X-100]. The cell proteins were extracted during 15 min. using a rotary shaker and were subsequently centrifuged at 14000xg during 15 min. All of the aforementioned steps were carried out at 4°C. After centrifuging, Laemmli buffer was added to the supernatants obtained. The total cell extracts were boiled during 10 min. and were loaded onto polyacrylamide gels with SDS (*sodium dodecyl sulphate*) (6% for NFAT and 10% for active CN or GFP). The proteins were separated by electrophoresis under reducing conditions. The protein gels were transferred to nitrocellulose membranes and were incubated during 1 hour at ambient temperature with a blocking solution [10% (weight/volume) of skimmed milk in PBS]. After a number of washes with PBS-T (0.1% of Tween-20 in PBS), the membranes were incubated during two hours with the respective primary antibody solution: monoclonal mouse anti-HA 12CA5 [0.05% (vol/vol)] in PBS-T with 1% of bovine serum albumin (BSA) for the detection of NFATc2; or polyclonal rabbit anti-GFP provided by Dr I. Crespo [0.1% (vol/vol)] in PBS-T with 5% of skimmed milk. The membranes were subsequently washed with PBS-T three times during 5 min. each time, and were incubated during 1 hour with the mouse anti-IgG antibodies made in goat (Pierce) or rabbit anti-IgG (Pierce) conjugated with peroxidase. After three washes with PBS-T and one additional wash with H₂O, the antibody bound to the membranes was detected by means of the ECL system (Amersham Biosciences).

### Immunofluorescences

The HeLa cells were transfected as described in the previous section using 1.2 µg of the GFP-peptide expression plasmids with 40 ng of pEF-BOS HA-NFATc2. Two hours before the stimulation, the culture medium was replaced with DMEM+1%FBS. After stimulating during 30 min. with calcium ionophore A23187 (1 µm) and CaCl₂ (3mM), the cells were fixed at ambient temperature during 10 min. with a 10% solution of formaldehyde in PBS containing 4% saccharose. Subsequently, they were washed three times with PBS and were permeabilised at ambient temperature during 10 min. with a solution of PBS with 0.25% of Triton X-100. The cells were subsequently incubated during 20 min. with a blocking buffer (10% BSA in PBS) and subsequently with a 1:500 solution of the 12CA5 antibody (anti-HA) during one hour. The subcellular localisation of the NFATS was detected using a fluorescence microscope (Axiovert-200, Zeiss) using a mouse anti-IgG conjugated with the Alexa 594 fluorochrome (Molecular Probes) as a secondary antibody. The percentage of cells with nuclear or cytosolic distribution of NFAT was calculated using not less than 130 positive cells per transfection.

### In vitro CN-NFAT binding assays.

The GST fusion proteins were expressed in the protease-deficient strain of E.coli BL2(DE3). They were purified using the *glutathione (GSH)-Sepharose B beads* reagent (Amersham Biosciences). The binding of CN to these GST fusion proteins was used to carry out pull-down tests, which were performed as explained below. A variable amount of the beads which contained the GST fusion proteins fused to the regulatory NFAT domains (1-2 µg) or to the peptides (10-30 µg). These were washed twice with the binding buffer (20mM Tris pH 8, 100mM NaCl, 1.5 mM CaCl₂, 6 mM MgCl₂, 1 mM PMSF, 1 µm DTT, 1 µg/ml of aprotinine, 1 µg/ml of pepstatin, 100 µM of benzamidine and 0.2% of Triton X-100) and were subsequently incubated during 30 min. in a rotary shaker at 4°C with a solution of 20 nM of Calcineurin (Sigma) plus 600 nM of Calmodulin (Sigma) in a binding buffer with or without the corresponding peptide. Subsequently, the samples were centrifuged briefly at 4°C. Laemmli buffer was added to the supernatants (unbound fraction), which were set aside for later analysis. The beads were washed five times with cold, recently prepared binding buffer and Laemmli buffer was added; the samples were boiled to separate the bound proteins (bound fraction). The bound and unbound fractions were loaded onto 10% SDS-polyacrylamide gels and were separated by electrophoresis under reducing conditions. The Calcineurin was detected by Western blot using an anti-CnA monoclonal mouse antibody (clone G182-1847, PharMingen). Where indicated, the amount of CN bound to the GST fusion proteins was quantified by densitometry using the Quantity One program (BIO-RAD).

In the assays in Figure 12, prior to the addition of CN and CaM, the immunophilins fused to GST were incubated with their corresponding immunosuppressor drug, CsA or FK506 (LC Laboratories). With regard to the experiments in Figure 11, CnA and CnB fused to GST (see section "Gene constructs") were used, purified from bacteria and subsequently digested with a commercial protease (PreScission protease, Amersham) in order to eliminate the GST portion. In these assays, the CnA sub-unit was detected by Western blot using the anti-CnA polyclonal rabbit antibody (Chemicon, whose epitope is found in the N-terminal region of CnA). For detection of the sub-unit CnB, a commercial monoclonal antibody was used (Upstate).

The peptides were synthesised at the Proteomics Service at the CSIC-UAM "Severo Ochoa" Molecular Biology Centre.

### Phosphatase activity assay of CN on RII substrate.

The effect of the peptides mentioned (Example 7, Figure 9) on the enzymatic activity of CN on the RII peptide was analysed using the "Biomol Green Calcineurin" assay kit by Biomol in accordance with the manufacturer's instructions. The combination of CsA (Sandoz) plus cyclophilin (CyP) (Sigma) was used to inhibit Calcineurin activity *in vitro.*

### Phosphatase activity assay of CN on pNPP (paranitrophenylphosphate).

The effect of the peptide (Example 8, Figure 10 and Table I) on the enzymatic activity of CN on pNPP was carried out in accordance with the protocol described (43) using 30 nM of human CN, expressed in bacteria and subsequently purified, incubating with 60 mM of pNPP during 30 minutes at 30°C in the presence of increasing amounts of the LxVPc1 peptide. In parallel, both the mutant LxVPc1 peptide, CsA or cyclophilin A (negative controls) and the combination cyclophilin A plus CsA (positive control) were used. The quantity of the product of the enzymatic reaction at basic pH (paranitrophenolate=PNP) was measured by absorbance at 405 nm, converting this numerical value to a concentration by means of interpolation from a straight-line PNP pattern.

In this application, especially in its figures, the amino acids are abbreviated using the one-letter codes accepted in the field, as shown below:

| | | |
|---|---|---|
| A= | Ala= | Alanine |
| C= | Cys= | Cystine |
| D= | Asp | Aspartic acid |
| E= | Glu= | Glutamic acid |
| F= | Phe= | Phenylalanine |
| G= | Gly= | Glycine |
| H= | His= | Histine |
| I= | Ile= | Isoleucine |
| K= | Lys= | Lysine |
| L= | Leu= | Leucine |
| M= | Met= | Methionine |
| N= | Asn= | Asparagine |
| P= | Pro= | Proline |
| Q= | Gln= | Glutamine |
| R= | Arg= | Arginine |
| S= | Ser= | Serine |
| T= | Thr= | Threonine |
| V= | Val= | Valine |
| W= | Trp= | Tryptophane |
| Y= | Tyr= | Tyrosine |

### References

1. Aramburu, J., Heitman, J. and Crabtree, G.R. (2004) EMBO Rep 5, 343.
2. Crabtree, G.R. and Olsen, E.N. (2002) Cell 109 Suppl, S67-79.
3. Horsley, V., and Pavlath, G.K. (2002) J Cell Biol 156, 771.
4. Lambrechts, D., and Carmeliet, P. (2004) Cell 118, 532.
5. Wilkins, B.J. and Molkentin, J.D. (2004) Biochem Biophys Res Commun 322, 1178.
6. Rao, A., Luo, C., and Hogan, P.G. (1997) Annu Rev Immunol 15, 707.
7. Klee, C.B., Ren, H. and Wang, X. (1998) J Biol Chem 273, 13367.
8. Stemmer, P.M. and Klee, C.B. (1994) Biochemistry 33, 6859.
9. Loh, C., Shaw, K.T., Carew, J., Viola, J.P.,Luo, C., Perrino, B.A. and Rao, A. (1996) J Biol Chem 271, 10884.
10. Wesselborg, S., Fruman, D.A., Sagoo, J.K., Bierer, B.E. and Burakoff, S.J. (1996) J Biol Chem 271, 1274.
11. García-Cozar, F.J., Okamura, H., Aramburu, J.F., Shaw, K.T., Pelletier, L., Showalter, R., Villafranca, E. and Rao, A. (1998) J Biol Chem 273, 23877.
12. Luo, C., Shaw, K.T., Raghavan, A., Aramburu, J., Garcia-Cozar, F., Perrino, B.A., Hogan, P.G. and Rao, A. (1996) Proc Natl Acad Sci USA 93, 8907.
13. Masuda, E.S., Liu, J., Imamura, R., Imai, S.I., Arai, K.I. and Arai, N. (1997) Mol Ce// Biol 17, 2066.
14. Shibasaki, F., Price, E.R., Milan, D. and McKeon, F. (1996) Nature 382, 370.
15. Aramburu, J., Garcia-Cozar, F., Raghavan, A., Okamura, H., Rao, A., and Hogan, P.G. (1998) Mol Cell 1, 627.
16. Chow, C.W., Rincon, M. and Davis, R.J (1999) Mol Ce// Biol 19, 2300.
17. Park, S., Uesugi, M. and Verdine, G.L. (2000) Proc Natl Acad Sci USA 97,7130.
18. Zhu, J., Shibasaki, F., Price, R., Guillemot, J.C., Yano, T., Dotsch, V., Wagner, G., Ferrara, P. and McKeon, F. (1998) Cell 93, 851.
19. Shaw, J.P., Utz, P.J., Durand, D.B., Toole, J.J., Emmel, E.A. and Crabtree, G.R. (1998) Science 241, 202.
20. Macian, F. (2005) Nat Rev Immunol 5, 472.
21. Macian, F., Garcia-Cozar, F., Im, S.H., Horton, H.F., Byrne, M.C. and Rao, A. (2002) Cell 109, 719.
22. Macian, F., López-Rodríguez, C. and Rao, A. (2001) Oncogene 20, 2476.
23. Serfling, E., Berberich-Siebelt, F., Avots, A., Chuvpilo, S., Klein-Hessling, S., Jha, M.K., Kondo, E., Pagel, P., Schulze-Luehrmann, J. and Palmetshofer, A. (2004) Int J Biochem Cell Biol 36, 1166.
24. Liu, J., Farmer, J.D. Jr., Lane, W.S., Friedman, J., Weissman, I. and Schreiber, S.L. (1991) Cell 66, 807.
25. Crabtree, G.R. and Clipstone, N.A. (1994) Annu Rev Biochem 63, 1045.
26. Liu, J. (1993) Immunol Today 14, 290.
27. Rao, A. (1994) Immunol Today 15, 274.
28. Kiani, A., Rao, A. and Aramburu, J. (2000) Immunity 12, 359.
29. Aramburu, J., Yaffe, M.B., López-Rodríguez, C., Cantley, L.C., Hogan, P.G. and Rao, A. (1999) Science 285, 2129.
30. Martinez-Martinez, S. and Redondo, J.M. (2004) Curr Med Chem 11, 997.
31. Halestrap et al. (1990) Biochem J 268, 153
32. Clarke et al. (2002) J Biol Chem 277, 34793.
33. Hojo et al. (1999) Nature 397: 530.
34. Sambrook et al. Molecular cloning, a Laboratory Manual 2nd ed., Cold Spring Harbor Laboratory Press, New York, 1989 vol 1-3.
35. Ralph et al. (2006) Clin Sci (Lond) 110, 37.
36. Swanson et al. (1992) Proc. Natl. Acad. Sci. USA 89, 3741.
37. Etzkorn et al. (1994) Biochemistry 33, 2380.
38. Ke and Huai (2003) Biochem Biophys Res Comm 311, 1095.
39. Gómez del Arco (2000) J Biol Chem 275, 13872.
40. Mondragon et al. (1997) Biochemistry 36, 4934.
41. Bultnyck et al. (2001) Biochem J 354, 413.
42. Bram et al. (1993) Mol Cell Biol. 13, 4760.
43. Sagoo et al. (1996) Biochem J 320, 879.

SEQUENCE LIST

<110> CSIC/CNIC

<120> Selective peptides that inhibit the biological activity of calcineurin

<130> Csic/Cnic

<160> 23

<170> PatentIn version 3.3

<210> 1
<211> 62
<212> DNA
<213> VIVITfw

<220>
<221> misc_feature
<222> (1)..(62)
<223> Codifying nucleotide sequence of the Vivit peptide

<400> 1
tcgagcatgg caggtccaca tccagtcatc gttatcaccg gtccacatga ggaaggcgga 60

cc 62

<210> 2
<211> 62
<212> DNA
<213> VIVITrev

<220>
<221> misc_feature
<222> (1)..(62)
<223> Complementary nucleotide sequence of the Vivit peptide.

<400> 2
catgggtccg ccttcctcat gtggaccggt gataacgatg actggatgtg gacctgccat 60

gc 62

<210> 3

<211> 63
<212> DNA
<213> PxIxITc1fw

<220>
<221> misc_feature
<222> (1)..(63)
<223> Codifying nucleotide sequence of the PxIxITc1 peptide.
<400> 3
agcttggcca ccatgccggc gttggaaagc ccacggatag agattacgag ttgcttggga 60

ttg 63

<210> 4
<211> 63
<212> DNA
<213> PxIxITc1rev

<220>
<221> misc_feature
<222> (1)..(63)
<223> Complementary nucleotide sequence of the PxIxITc1 peptide

<400> 4
gatccaatcc caagcaactc gtaatctcta tccgtgggct ttccaacgcc ggcatggtgg 60

cca 63

<210> 5
<211> 53
<212> DNA
<213> LxVPc1fw

<220>
<221> misc_feature
<222> (1)..(53)
<223> Codifying nucleotide sequence of the LxVPc1 peptide

<400> 5
tcgagcgatc agtacttggc cgtaccacag catccgtatc aatgggctaa ggg 53

<210> 6

<211>  53
<212>  DNA
<213>  LxVPc1rev

<220>
<221>  misc_feature
<222>  (1)..(53)
<223> Complementary nucleotide sequence of the LxVPc1 peptide

<400>  6
aattccctta gcccattgat acggatgctg tggtacggcc aagtactgat cgc          53

<210>  7
<211>  60
<212>  DNA
<213>  mutLxVPc1fw

<220>
<221>  misc_feature
<222>  (1)..(60)
<223>  Codifying nucleotide sequence of the mutLxVPc1 peptide

<400>  7
agcttggcca ccatggatca gtatgcggca gctgcccagc atccgtatca atgggcgaag          60

<210>  8
<211>  60
<212>  DNA
<213>  mutLxVPc1rev

<220>
<221>  misc_feature
<222>  (1)..(60)
<223>  Complementary nucleotide sequence of the mutLxVPc1 peptide

<400>  8
gatccttcgc ccattgatac ggatgctggg cagctgccgc atactgatcc atggtggcca          60

<210>  9
<211>  63
<212>  DNA
<213>  PxIxITc2fw

<220>
<221> misc_feature
<222> (1)..(63)
<223> Codifying nucleotide sequence of the PxIxITc2 peptide

<400> 9
gatctgccac catggcgagt gggttgagcc cgaggataga gataacgccg agtcatgagt 60

tag 63

<210> 10
<211> 63
<212> DNA
<213> PxIxITc2rev

<220>
<221> misc_feature
<222> (1)..(63)
<223> Complementary nucleotide sequence of the PxIxITc2 peptide

<400> 10
gatcctaact catgactcgg cgttatctct atcctcgggc tcaacccact cgccatggtg 60

gca 63

<210> 11
<211> 60
<212> DNA
<213> LxVPc2fw

<220>
<221> misc_feature
<222> (1)..(60)
<223> Codifying nucleotide sequence of the LxVPc2 peptide

<400> 11
agcttggcca ccatggagag catcctgcta gtacctccga cctggcctaa gccgttggtg 60

<210> 12
<211> 60
<212> DNA

<213>  LxVPc2rev

<220>
<221>  misc_feature
<222>  (1)..(60)
<223>  Complementary nucleotide sequence of the LxVPc2 peptide

<400>  12
gatccaccaa cggcttaggc caggtcggag gtactagcag gatgctctcc atggtggcca          60

<210>  13
<211>  16
<212>  PRT
<213>  VIVIT

<220>
<221>  PEPTIDE
<222>  (1)..(16)
<223>  aa. sequence of the Vivit peptide

<400>  13

Mct Ala Gly Pro His Pro Val Ilc Val Ilc Thr Gly Pro His Glu Glu
1               5                   10                  15

<210>  14
<211>  16
<212>  PRT
<213>  PxIxITc1ç

<220>
<221>  PEPTIDE
<222>  (1)..(16)
<223>  aa. sequence of the PxIxITc1 peptide

<400>  14

Pro Ala Leu Glu Ser Pro Arg Ile Glu Ile Thr Ser Cys Leu Gly Leu
1               5                   10                  15

<210>  15
<211>  16

<212>  PRT
<213>  PxIxITc2

<220>
<221>  PEPTIDE
<222>  (1)..(16)
<223>  aa. sequence of the PxIxITc2 peptide

<400>  15

Ala Ser Gly Leu Ser Pro Arg Ile Glu Ile Thr Pro Ser His Glu Leu
1               5                   10                  15

<210>  16
<211>  16
<212>  PRT
<213>  PxIxITc3

<220>
<221>  PEPTIDE
<222>  (1)..(16)
<223>  aa. sequence of the PxIxITc3 peptide

<400>  16

Lys Pro Phe Glu Cys Pro Ser Ile Gln Ile Thr Ser Ile Ser Pro Asn
1               5                   10                  15

<210>  17
<211>  16
<212>  PRT
<213>  PxIxITc4

<220>
<221>  PEPTIDE
<222>  (1)..(16)
<223>  aa. sequence of the PxIxITc4 peptide

<400>  17

Arg Val Leu Glu Cys Pro Ser Ile Arg Ile Thr Ser Ile Ser Pro Thr
1               5                   10                  15

<210> 18
<211> 15
<212> PRT
<213> LxVPc1

<220>
<221> PEPTIDE
<222> (1)..(15)
<223> aa. sequence of the LxVPc1 peptide

<400> 18

Asp Gln Tyr Leu Ala Val Pro Gln His Pro Tyr Gln Trp Ala Lys
1               5                   10                  15

<210> 19
<211> 15
<212> PRT
<213> mutLxVPc1

<220>
<221> PEPTIDE
<222> (1)..(15)
<223> aa. sequence of the mutLxVPc1 peptide

<400> 19

Asp Gln Tyr Ala Ala Ala Ala Gln His Pro Tyr Gln Trp Ala Lys
1               5                   10                  15

<210> 20
<211> 15
<212> PRT
<213> LxVPc2

<220>
<221> PEPTIDE
<222> (1)..(15)
<223> aa. sequence of the LxVPc2 peptide

<400> 20

Glu Ser Ile Leu Leu Val Pro Pro Thr Trp Pro Lys Pro Leu Val
1               5                   10                  15

<210>  21
<211>  15
<212>  PRT
<213>  LxVPc3

<220>
<221>  PEPTIDE
<222>  (1)..(15)
<223>  aa. sequence of the LxVPc3 peptide

<400>  21

Asp Gln Phe Leu Ser Val Pro Ser Pro Phe Thr Trp Ser Lys Pro
1               5                   10                  15

<210>  22
<211>  15
<212>  PRT
<213>  LxVPc4

<220>
<221>  PEPTIDE
<222>  (1)..(15)
<223>  aa. sequence of the LxVPc4 peptide

<400>  22

Met Asp Tyr Leu Ala Val Pro Ser Pro Leu Ala Trp Ser Lys Ala
1               5                   10                  15

<210>  23
<211>  16
<212>  PRT
<213>  SCRAMBLED

<220>
<221>  PEPTIDE
<222>  (1)..(16)
<223>  aa. sequence of the Scrambled peptide

<400> 13

Met Val Gly Ile Pro Val Ala Ile His Gly Thr Pro Pro His Glu Glu
1               5                   10                  15

## Claims

1. An amino acid sequence, for the selective inhibition of the Calcineurin (CN) signalling pathway on the NFAT transcription factors (CN-NFAT), **characterised in that** it comprises the amino acid sequence:
R₁-L-R₂-V-P-R₃,
wherein R1 is an aromatic amino acid of the Tyrosine or Phenylalanine type; R2 is an amino acid of the Alanine or Serine type and R3 is not the amino acid Proline.

2. An amino acid sequence, according to claim 1, **characterised in that** said transcription factors are, at least, NFATc1 and NFATc2.

3. An amino acid sequence, according to either of claims 1 or 2, **characterised in that** it is comprised of an amino acid sequence belonging to the following group:
a) Amino acid sequence SEQ ID NO 18.
b) Amino acid sequence analogous to the sequence in a), and
c) Amino acid sequence which comprises any sequence belonging to a) or b).

4. An amino acid sequence, according to either of claims 1 or 2, **characterised in that** it is comprised of an amino acid sequence belonging to the following group:
a. Amino acid sequence SEQ ID NO 21.
b. Amino acid sequence analogous to the sequence in a), and
c. Amino acid sequence which comprises any sequence belonging to a) or b).

5. An amino acid sequence, according to either of claims 1 or 2, **characterised in that** it is comprised of an amino acid sequence belonging to the following group:
a. Amino acid sequence SEQ ID NO 22.
b. Amino acid sequence analogous to the sequence in a), and
c. Amino acid sequence which comprises any sequence belonging to a) or b).

6. A nucleotide sequence capable of encoding a sequence of amino acids in accordance with any of claims 1 to 5.

7. A nucleotide sequence according to claim 6, where said sequence is selected from one of the following groups:
a. Nucleotide sequence SEQ ID NO 5 and/or its complementary nucleotide sequence SEQ ID NO 6,
b. Encoding nucleotide sequence for the amino acid sequence SEQ ID NO 21,
c. Encoding nucleotide sequence for the amino acid sequence SEQ ID NO 22,
d. Nucleotide sequence analogous to sequence a) or b) or c)
e. Nucleotide sequence which comprises any sequence belonging to a), b), c) or d).

8. A nucleotide sequence according to claim 7, where said sequence is SEQ ID NO 5 and/or SEQ ID NO 6.

9. A genetic construct **characterised in that** it comprises a nucleotide sequence according to any of claims 6 to 8.

10. A genetic construct, according to claim 9, **characterised in that** it also comprises a peptide or peptidic sequence-encoding nucleotide sequence which permits the isolation or detection of the aforementioned peptide.

11. A gene expression vector which comprises a nucleotide sequence according to any of claims 6 to 8 or the gene construct according to claims 9 and 10.

12. A gene expression vector, according to claim 11, where said vector is a plasmid.

13. A transformed cell which contains a nucleotide sequence according to any of claims 6 to 8, the gene construct according to claims 9 and 10, or the expression vector according to either of claims 11 and 12.

14. A procedure for the identification of new pharmaceutical compounds which are capable of inhibiting the biological action of Calcineurin on the NFATc1 and NFATc2 transcription factors, which comprises the use of a peptide according to any of claims 1 to 5, a nucleotide sequence according to any of claims 6 to 8, the gene construct according to claims 9 and 10, the expression vector according to either of claims 11 and 12, or the transformed cell according to claim 13.

15. A procedure for the identification of new compounds, according to claim 14, where said compounds are selective antagonists of the biological activity of Calcineurin A.

16. A procedure for the identification of new compounds, according to claim 14, where said procedure is an in vitro protein interaction or binding assay.

17. A procedure for the identification of new compounds, according to claim 14, where said procedure is an in vivo protein interaction or binding assay.

18. The use of an element or blend of elements according to claims 1 to 13 in the preparation of a drug for the treatment or prophylaxis of diseases associated with Calcineurin activation.

19. A pharmaceutical composition useful for the treatment of diseases,
**characterised in that** it comprises an element or blend of elements according to claims 1 to 13 with, optionally, one or more adjuvants and/or pharmaceutically acceptable vehicles.

20. A pharmaceutical composition, according to claim 19, where said element is an amino acid sequence according to any of claims 1 to 5.

21. A pharmaceutical composition, according to claim 19, where said element is a nucleotide sequence according to any of claims 6 to 8.

22. A pharmaceutical composition, according to claim 19, where said element is a genetic construct according to either of claims 9 or 10.

23. A pharmaceutical composition, according to claim 19, where said element is an expression vector according to either of claims 11 or 12.

24. A pharmaceutical composition, according to claim 19, where said element is a transformed cell according to claim 13.

25. Use of the pharmaceutical composition, according to claims 19 to 24, for the treatment or prophylaxis of diseases associated with Calcineurin activation.

26. Use of the pharmaceutical composition, according to claims 19 to 24, for the treatment of diseases associated with T-lymphocyte activation and which belong to the following group: auto-immune diseases, inflammation, allergy or transplant rejection situations.
